# EUROPEAN PATENT APPLICATION

(11) **EP 2 450 699 A1**
(43) Date of publication of application: **09.05.2012**
(21) Application number: 10794086.8
(22) Date of filing: 25.06.2010
(51) Int. Cl.: G01N 27/26, G01N 27/28, G01N 27/327, G01N 27/416

(54) **ANALYSIS DEVICE AND ANALYSIS METHOD**

(30) Priority: 30.06.2009 JP 2009156446
(71) Applicant: Arkray, Inc., Minami-ku Kyoto-shi Kyoto 601-8045 (JP)
(72) Inventor: NAKANISHI, Hiroyuki, Kyoto-shi Kyoto 602-0008 (JP); UEHATA, Yoshiharu, Kyoto-shi Kyoto 602-0008 (JP); KAWANISHI, Masako, Kyoto-shi Kyoto 602-0008 (JP); MURASE, Yosuke, Kyoto-shi Kyoto 602-0008 (JP); TSUKADA, Masashi, Kyoto-shi Kyoto 602-0008 (JP)
(74) Representative: MacDougall, Alan John Shaw
(86) International application number: PCT/JP2010/060896
(87) International publication number: WO 2011/001917

(57) **Abstract**

Provided is an analysis device or an analysis method, by which highly reliable analysis results can be obtained even in the circumstances where environment temperature changes, while reducing load on the user. The analysis device (1) is provided with a determining means (13) which determines whether environmental temperature measured by means of a temperature measuring means (6) is within a predetermined temperature range or not. The determining means (13) is so configured as to determine whether the environmental temperature is within the predetermined temperature range or not, even in the circumstances where information relating to a target substance in a sample cannot be obtained from the analysis device.

## Description

### TECHNICAL FIELD

The present invention relates to an analysis device (analysis apparatus) and an analysis method for analyzing a specified component contained in a sample by using an analysis instrument.

### BACKGROUND ART

A method for measuring the blood glucose level (blood sugar level) has been hitherto known. In this method, a blood glucose level measuring apparatus and an analysis instrument are used in combination, and thus the blood glucose level is measured automatically by means of the blood glucose level measuring apparatus. The analysis instrument is exemplified by an analysis instrument which is used while being installed to the blood glucose level measuring apparatus and an analysis instrument which is used while being held or subjected to detention subcutaneously. In general, a biosensor, which utilizes the enzyme reaction, is used therefor.

As for the enzyme, the enzyme activity is fluctuated depending on the reaction temperature. Therefore, in the case of a method in which the biosensor is used, the environmental temperature is measured as the reaction temperature, and the calculated value is corrected depending on the environmental temperature. The temperature correction is performed by using the temperature correction data which indicates the temperature dependency and which is empirically obtained beforehand. The temperature correction data makes it possible to determine the correction amount and the correction coefficient on the basis of the temperature difference with respect to the ordinary temperature, for example, by using the ordinary temperature (for example, 25°C) as the reference. The influence, which is exerted by the environmental temperature, can be canceled on the basis of the correction amount. The temperature correction data as described above is usually determined for a predetermined temperature range (for example, 10 to 40°C).

However, when the biosensor is used while being installed to the blood glucose level measuring apparatus, the temperature correction as described above is performed as one of the blood glucose level calculating operations after blood is spotted on the biosensor and the analysis of blood is started. For this reason, it is impossible for a user to grasp whether or not the environmental temperature is within a temperature range in which the temperature correction can be appropriately performed (within a temperature range in which the temperature correction data is generated), until the blood glucose level calculating operations are completed. Therefore, if the environmental temperature is without the temperature range in which the temperature correction can be appropriately performed, the biosensor is consumed uselessly, in spite of such a situation that the blood glucose level cannot be measured appropriately.

On the other hand, in order to obtain the reliable measured value, it is necessary that the remeasurement should be repeated until the environmental temperature is settled within the temperature range in which the temperature correction can be appropriately performed. In particular, when the analysis apparatus is used in a situation in which the temperature change is intense, for example, when the analysis apparatus is used while being moved from the cold outdoor to the warm indoor, then a relatively long time is required until the environmental temperature becomes a temperature at which the correction can be performed (for example, 10°C). In this case, a user is forced to perform the complicated operation, because it is necessary to repeatedly install and remove the biosensor. Further, the biosensor is uselessly consumed in many cases as well.

In the case of the measured value obtained at any environmental temperature at which the temperature correction is not performed appropriately, the reliability and the reproducibility of the measured value are lowered. For example, in the case of the method in which the biosensor is used while being installed to the blood glucose level measuring apparatus, the temperature of the enzyme of the biosensor tends to be affected by the environmental temperature itself or the change of the environmental temperature. Therefore, the enzyme activity is affected by the measurement environment and/or the change thereof, and the reliability and the reproducibility of the measured value are lowered in some cases.

On the other hand, when the biosensor is used while being held subcutaneously, the biosensor is usually held subcutaneously so that the portion of the biosensor, at which the enzyme is immobilized, is positioned at a subcutaneous portion disposed within 10 mm. The subcutaneous range as described above resides in such a portion that the influence is exerted by the change of the temperature of the outside air and the temperature change is relatively large. Therefore, also in the case of the blood glucose level measuring apparatus which is used while holding the biosensor subcutaneously, the enzyme activity of the biosensor is affected by the temperature change caused, for example, by the change of the measurement place, and the reliability and the reproducibility of the measured value are lowered in some cases.

Further, even when the biosensor is used while being held subcutaneously, the reliability and the reproducibility are lowered in relation to the measured value obtained in the environment in which the temperature correction is not performed appropriately. Therefore, it is impossible to use the measured value obtained within the concerning time. When the voltage is applied to the biosensor in order to obtain the measured value, the biosensor is deteriorated corresponding thereto.
Therefore, when any unusable measured value is obtained, the service life of the biosensor is uselessly reduced by an amount of time in which the voltage is applied in order to obtain the unusable measured value.

Further, when the reliability of the measured value is low, it is also necessary that the user should be informed of the fact (fact that the temperature is without the usable temperature), for example, by displaying the fact on a display unit such as LCD or the like of the blood glucose level measuring apparatus (see, for example, Patent Documents 1 and 2).

### PRECEDING TECHNICAL DOCUMENTS

### Patent Documents:

Patent Document 1: JP8-503304W;
Patent Document 2: JP2003-42995A.

### SUMMARY OF THE INVENTION

### Task to Be Solved by the Invention:

An object of the present invention is to provide an analysis apparatus and an analysis method in which a highly reliable analysis result is obtained even in such a situation that the environmental temperature is changed, while mitigating the load exerted on a user.

### Solution for the Task:

The present invention relates to an analysis apparatus for obtaining information with respect to an objective substance contained in a sample. The analysis apparatus comprises temperature measuring means which measures an environmental temperature; storage means which stores temperature correction data corresponding to the environmental temperature in relation to a predetermined temperature range; calculating means which calculates an analysis value on the basis of the temperature correction data and the information with respect to the objective substance contained in the sample; and determining means which determines whether or not the environmental temperature is within the temperature range.

The determining means may determine whether or not the environmental temperature is within the temperature range on the basis of one environmental temperature measured by the temperature measuring means. Alternatively, the determining means may determine whether or not the temperature is within a temperature range in which the temperature correction data is applicable, depending on whether or not an amount of change per predetermined time of the environmental temperature measured a plurality of times by the temperature measuring means is within a predetermined range. Of course, the determining means may determine whether or not the temperature is within the temperature range in which the temperature correction data is applicable, if the amounts of change of the plurality of environmental temperatures are within the predetermined range, if it is determined that one environmental temperature is within the temperature range.

The determining means determines whether or not the environmental temperature is within the temperature range even in such a situation that the information with respect to the objective substance contained in the sample is not obtained from the analysis apparatus. The situation, in which the information with respect to the objective substance contained in the sample is not obtained from the analysis apparatus, is herein, for example, such a situation that the sample is not supplied to the analysis apparatus. The "information with respect to the objective substance contained in the sample" is, for example, any numerical information for which a response current value (or a voltage value obtained by the conversion from the response current value) can be exemplified. The "analysis value" can be exemplified by "a corrected response current value or a corrected voltage value" or "a glucose concentration in blood or interstitial fluid".

The determining means may determine whether or not the temperature is within a temperature range in which the temperature correction data is applicable, depending on an amount of change of the environmental temperature per predetermined time.

In the present invention, an additional temperature measuring means, which measures the environmental temperature, may be provided distinctly from the temperature measuring means. Further, the determining means may be constructed to determine whether or not the temperature is within a temperature range in which the temperature correction data is applicable, on the basis of a difference between the environmental temperature which is measured by the temperature measuring means and the environmental temperature which is measured by the additional temperature measuring means.

The temperature measuring means may be provided at a portion at which it is possible to measure a temperature in the vicinity of a portion which outputs the information with respect to the objective substance contained in the sample. On the other hand, the additional temperature measuring means may be provided at a portion separated from a portion which outputs the information with respect to the objective substance contained in the sample as compared with the temperature measuring means. The additional temperature measuring means measures, for example, an outside air temperature or an epidermal temperature.

The determining means may be also constructed to determine again whether or not the environmental temperature is within a temperature range in which the temperature correction data is applicable, after a certain time elapses, if it is determined that the environmental temperature is without the temperature range in which the temperature correction data is applicable.

The analysis apparatus may further comprise a main power source; wherein the determining means may stop the main power source or perform electric power saving after a certain time elapses, if it is determined that the environmental temperature is without the temperature range in which the temperature correction data is applicable.

It is preferable that the analysis apparatus of the present invention further comprises informing means which informs that the environmental temperature is within the temperature range in which the temperature correction data is applicable or that the environmental temperature is without the temperature range in which the temperature correction data is applicable, on the basis of a determination result obtained by the determining means.

The analysis apparatus of the present invention may further comprise operating means which is operated to inform a user of a determination result obtained by the determining means, by means of the informing means. The operating means can be exemplified by an operation button.

An apparatus, which can be held in a body and which is capable of continuously analyzing the sample, may be used as the analysis apparatus. It is noted that the term "in a body" may include "subcutaneously".

In another aspect, the present invention relates to an analysis method for obtaining information with respect to an objective substance contained in a sample. The analysis method comprises a temperature measuring step of measuring an environmental temperature; a storing step of storing temperature correction data corresponding to the environmental temperature in relation to a predetermined temperature range; a calculating step of calculating an analysis value on the basis of the temperature correction data and the information with respect to the objective substance contained in the sample; and a determining step of determining whether or not the environmental temperature is within the temperature range; wherein it is determined in the determining step whether or not the environmental temperature is within the temperature range even in such a situation that the information with respect to the objective substance contained in the sample is not obtained from the analysis step.

### EFFECT OF THE INVENTION

According to the present invention, it is possible to provide the analysis apparatus and the analysis method in which the highly reliable analysis result is obtained even in such a situation that the environmental temperature is changed, while reducing the load exerted on a user.

### BRIEF DESCRIPTION OF THE DRAWINGS

Fig. 1 illustrates a perspective view illustrating an example of an entire blood glucose level measuring apparatus according to the present invention.
Fig. 2 illustrates a sectional view illustrating main parts of the blood glucose level measuring apparatus illustrated in Fig. 1.
Fig. 3 illustrates a perspective view illustrating an example of an entire biosensor used for the blood glucose level measuring apparatus illustrated in Fig. 1.
Fig. 4 illustrates a sectional view illustrating the biosensor illustrated in Fig. 3.
Fig. 5 illustrates an exploded perspective view illustrating those disposed in relation to a connector unit in the blood glucose level measuring apparatus illustrated in Fig. 1.
Fig. 6 illustrates a block diagram of the blood glucose level measuring apparatus illustrated in Fig. 1.
Fig. 7 illustrates a sectional view corresponding to Fig. 2, illustrating another example of a blood glucose level measuring apparatus according to the present invention.
Fig. 8 illustrates a block diagram of the blood glucose level measuring apparatus illustrated in Fig. 7.
Fig. 9 illustrates a sectional view corresponding to Fig. 2, illustrating still another example of a blood glucose level measuring apparatus according to the present invention.
Fig. 10 illustrates a sectional view illustrating an example of a glucose continuous measuring apparatus for carrying out a continuous measuring method for continuously measuring glucose.
Fig. 11 illustrates a perspective view illustrating an entire glucose sensor for the glucose continuous measuring apparatus illustrated in Fig. 10, together with an enlarged view illustrating main parts.
Fig. 12 illustrates a perspective view illustrating the entire glucose sensor for the glucose continuous measuring apparatus illustrated in Fig. 10, together with an enlarged view illustrating main parts.
Fig. 13 illustrates a block diagram illustrating a schematic arrangement of the glucose continuous measuring apparatus illustrated in Fig. 10.
Fig. 14 illustrates sectional views illustrating other examples of glucose continuous measuring apparatuses according to the present invention.

### MODE FOR CARRYING OUT THE INVENTION

An explanation will be made below with reference to the drawings with respect to a blood glucose level measuring apparatus as an example of the analysis apparatus and the analysis method according to the present invention.

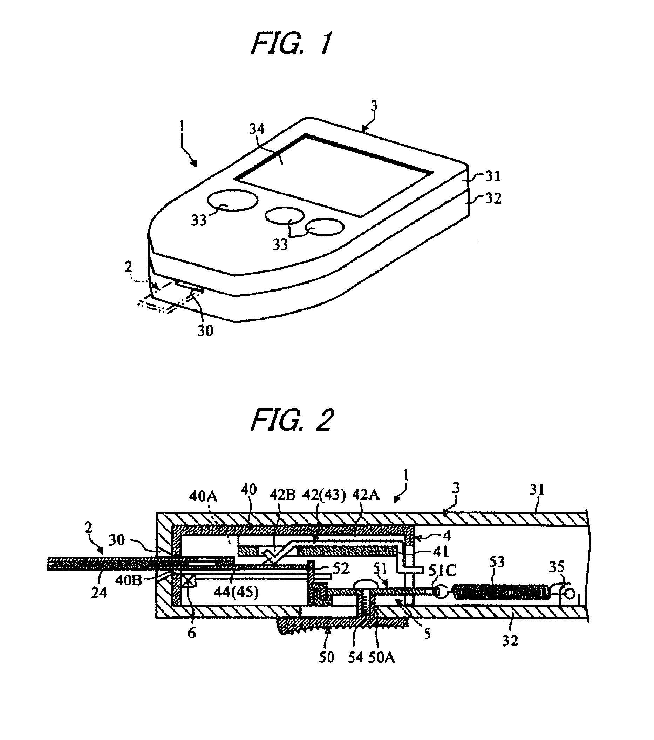

The blood glucose level (blood sugar level) measuring apparatus 1 illustrated in Figs. 1 and 2 is provided to analyze a sample in accordance with an electrochemical technique by using a biosensor 2. The blood glucose level measuring apparatus 1 is constructed as a portable type capable of being carried or transported. The blood glucose level measuring apparatus 1 is provided with a housing 3, a connector unit 4, a discarding mechanism 5, and a temperature measuring unit 6.

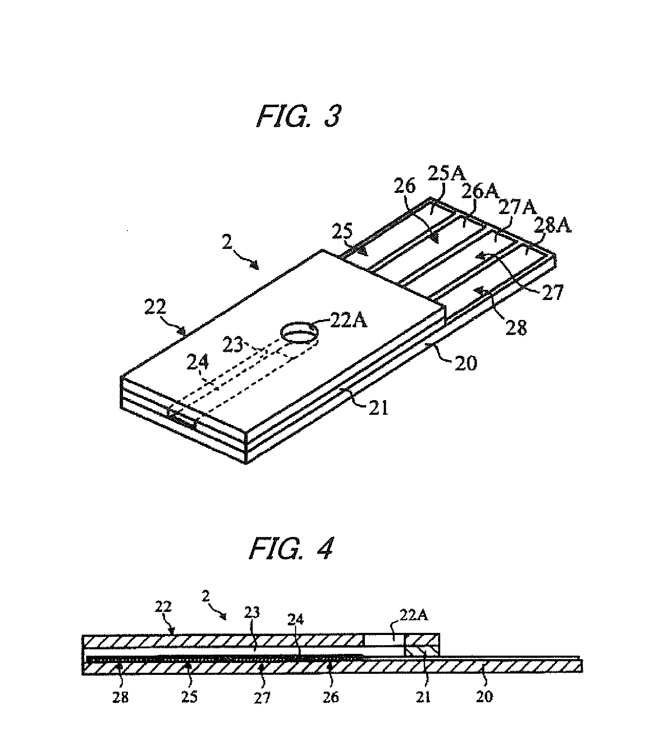

As illustrated in Figs. 3 and 4, the biosensor 2, which is used for the blood glucose level measuring apparatus 1, is constructed to be disposable. The biosensor 2 is formed to have a flat plate-shaped form as a whole. The biosensor 2 is constructed such that a cover 22 is joined to a substrate 20 having a substantially elongated rectangular shape by the aid of a spacer 21 intervening therebetween. A capillary 23, which extends in the longitudinal direction of the substrate 20, is defined by the respective elements 20 to 22.

The capillary 23 is provided in order that blood is moved toward a gas discharge port 22A of the cover 22 as described later on by utilizing the capillary phenomenon and introduced blood is retained. A reagent layer 24 is provided in the capillary 23. The reagent layer 24 includes, for example, an electron transport (electron transfer) substance (complex such as [Ru(NH₃)₆]Cl₃, K₃[Fe(CN)₆] or the like) and oxidoreductase (glucose oxidase (GOD) or glucose dehydrogenase (GDH)).

The spacer 21 is provided to define the distance between the substrate 20 and the cover 22, i.e., the height dimension of the capillary 23. The spacer 21 is constructed, for example, by a double-sided adhesive tape.

The cover 22 has the gas discharge port 22A for discharging the gas contained in the capillary 23 to the outside. The cover 22 is formed of a thermoplastic resin having the high wettability including, for example, vinylon and highly crystallized PVA.

The substrate 20 is formed of an insulative resin material to have a shape larger than that of the cover 22. A plurality of electrodes 25, 26, 27, 28 (four in the drawing) are formed on the upper surface of the substrate 20.

The plurality of electrodes 25 to 28 include at least a working electrode and a counter electrode for applying the voltage to the blood introduced into the capillary 23. The plurality of electrodes 25 to 28 further include a detecting electrode for detecting the fact that the blood is supplied to the inside of the capillary 23, an information output electrode for outputting the information with respect to the biosensor 2 (for example, the date of production of the biosensor, the production factory, the lot No., and the sensor sensitivity (type of the calibration curve to be selected)), and a static electricity countermeasure electrode for the countermeasure against the static electricity. The plurality of electrodes 25 to 28 may include any electrode having any other function in addition to the detecting electrode, the information output electrode, and the static electricity countermeasure electrode, or in place of at least one of the electrodes as described above.

It is a matter of course that the number of the plurality of electrodes is not limited to four, which can be subjected to the design change depending on the purpose. Further, the form of the plurality of electrodes can be also subjected to the design change in various ways. For example, Japanese Application Publication No. 04-357452 discloses that two or four electrodes are provided, and the electrodes are bent so that the respective electrodes are not overlapped with each other in an identical plane. For example, Japanese application publication No.08-320304 discloses that three electrodes are provided, and the electrodes are bent so that the respective electrodes are not overlapped with each other in an identical plane. For example, domestic re-publication of PCT international publication for patent application No.2004/051249 discloses that two or three electrodes are provided, and the electrodes are bent so that the respective electrodes are not overlapped with each other in an identical plane. Similarly thereto, also in this embodiment, it is possible to provide two or more electrodes. Further, the electrodes may be bent so that the respective electrodes are not overlapped with each other in an identical plane.

As illustrated in Figs. 1 and 2, the housing 3 defines the appearance shape of the blood glucose level measuring apparatus 1, and the housing 3 is provided to accommodate the various elements represented by the connector unit 4, the discarding mechanism 5, and the temperature measuring unit 6. The housing 3 has a sensor insertion port 30. Further, the housing 3 is formed to be hollow by casings 31, 32.

The casing 31 is provided with a plurality of operation buttons 33, and a display panel 34. An operation lever 50 of the discarding mechanism 5 is movably retained by the casing 32 as described later on.

The plurality of operation buttons 33 are provided to generate signals in order to perform the operations required to analyze blood, in addition to various types of setting (for example, setting of the analysis condition and the input of ID of an examinee).
The operation button 33 may be used to confirm whether or not the environmental temperature is within a usable temperature of the blood glucose level measuring apparatus 1.

The analysis result and the fact that any error arises are displayed on the display panel 34. Further, the display panel 34 is provided to display, for example, the operation situation and the operation procedure during the setting. The error herein includes, for example, the insufficient supply of blood to the biosensor 2, the erroneous operation of the blood glucose level measuring apparatus 1, the erroneous calculation in the blood glucose level measuring apparatus 1, the operation mistake and the setting mistake by a user, and the case in which the environmental temperature is without the usable temperature of the blood glucose level measuring apparatus 1.

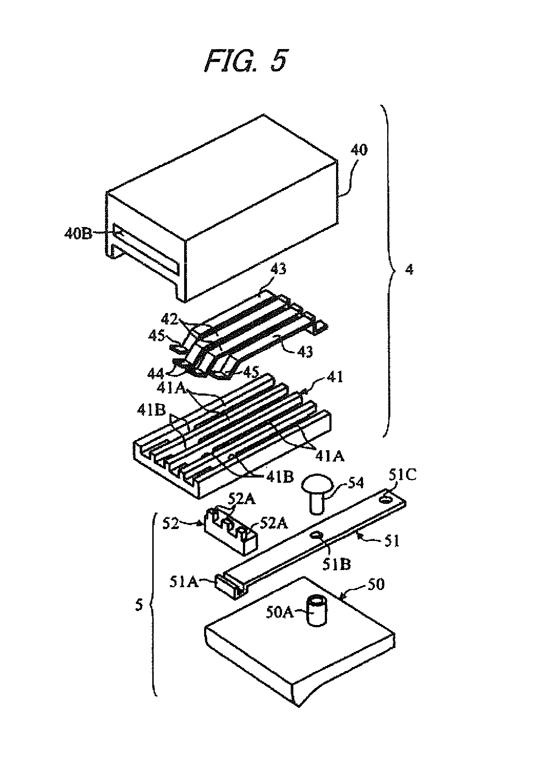

As illustrated in Figs. 2 and 5, the biosensor 2 is installed to the connector unit 4. The connector unit 4 has a case 40, a terminal base 41, and a plurality of terminals 42, 43 (four in the drawing).

The case 40 is provided to hold the plurality of terminals 42, 43 and the terminal base 41, and the case 40 is provided to hold the biosensor 2. The case 40 has a hollow portion 40A for holding the terminal base 41 and an opening 40B for introducing/withdrawing the biosensor 2.

The terminal base 41 is provided to fix the plurality of terminals 42, 43. The terminal base 41 has a plurality of slits 41A for accommodating the terminals 42, 43 and through-holes 41B for allowing forward end portions 44, 45 of the terminals 42, 43 to protrude.

The plurality of terminals 42, 43 are brought in contact with terminal portions 25A to 28A (see Fig. 3) of the plurality of electrodes 25 to 28 of the biosensor 2 when the biosensor 2 is installed to the connector unit 4 in order that the voltage is applied to the plurality of electrodes 25 to 28 and the current value (resistance value), which is provided in the concerning situation, is measured. The respective terminals 42, 43 have the forward end portions 44, 45 which are constructed as plate springs, and they also play such a role that the forward end portions 44, 45 allow the pressing force to act on the biosensor 2, and the biosensor 2 is appropriately retained in the connector unit 4, when the biosensor 2 is installed to the connector unit 4.

As illustrated in Fig. 2, the discarding mechanism 5 is provided to discard the biosensor 2 from the blood glucose level measuring apparatus 1 after the completion of the measurement of the blood glucose level. The discarding mechanism 5 is provided with an operation lever 50, a connecting plate 51, a slide block 52, and a coil spring 53.

The operation lever 50 is a portion which is operated in order to move the slide block 52. The operation lever 50 is reciprocatively movable with respect to the housing 3 in a state in which a part thereof is exposed from the housing 3 (casing 31). The operation lever 50 is fixed to the connecting plate 51 by using a screw 54 at a female screw portion 50A.

The connecting plate 51 is provided to connect the operation lever 50 and the slide block 52. The connecting plate 51 has a crank portion 51A and through-holes 51B, 51C. The crank portion 51A is a portion which is provided to fix the slide block 52. The through-hole 51B is a portion through which the screw 54 is inserted in order to fix the operation lever 50. The through-hole 51C is a portion at which one end of the coil spring 53 is fastened.

The slide block 52 is provided to move the biosensor 2 installed to the connector unit 4. The slide block 52 is moved in cooperation with the motion of the operation lever 50.

The coil spring 53 is provided to restore the connecting plate 51 as well as the operation lever 50 and the slide block 52 to the waiting positions. The coil spring 53 has one end which is fastened to the connecting plate 51 as described above and the other end which is fastened to a fixing portion 35 of the housing 3. Therefore, the coil spring 53 is elongated when the operation lever 50 is moved by allowing the load to act in the discarding direction on the operation lever 50. On the other hand, the coil spring 53 is shrunk when the operation lever 50 is released from the load having been allowed to act thereon, and the operation lever 50 and the slide block 52 are restored to the waiting positions.

The temperature measuring unit 6 is provided to measure the environmental temperature. The temperature measuring unit 6 is provided at a portion at which it is possible to measure the temperature in the vicinity of the reagent layer 24 of the biosensor 2. It is preferable that the temperature measuring unit 6 is provided at a position which is extremely near to the portion corresponding to the working electrode of the plurality of electrodes 25 to 28. In the example illustrated in the drawing, the temperature measuring unit 6 is provided at the position at which the environmental temperature can be measured in the vicinity of the working electrode in relation to the reagent layer 24 from the back surface side of the biosensor 2. Those usable as the temperature measuring unit 6 include, for example, a thermistor as well as various known devices.

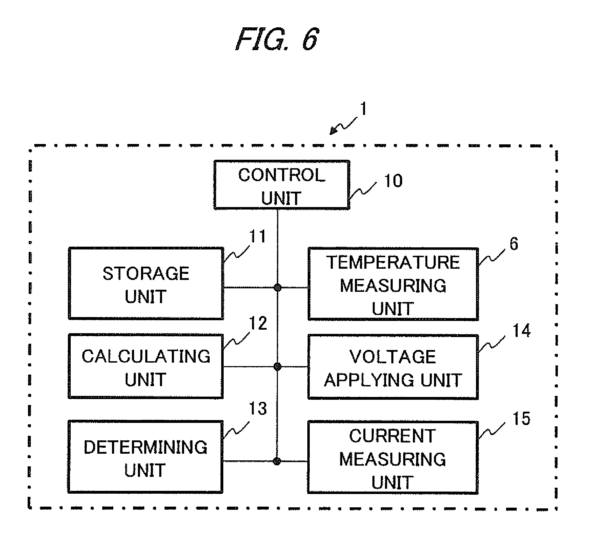

As illustrated in Fig. 6, the blood glucose level measuring apparatus 1 further comprises a control unit (controller) 10, a storage unit (storage) 11, a calculating unit (calculator) 12, a determining unit 13, a voltage applying unit 14, and a current measuring unit 15.

The control unit 10 controls the operations of the respective parts of the blood glucose level measuring apparatus 1. For example, the control unit 10 controls the display operation performed on the display panel 34, the determining operation performed by the determining unit 13, the voltage applying operation performed by the voltage applying unit 14, and the calculating operation for calculating the blood glucose level by the calculating unit 12.

The storage unit 11 stores the program required to calculate the blood glucose level, including, for example, the correlation data between the current value (voltage value) and the blood glucose level and the temperature correction data corresponding to the environmental temperature. The temperature correction data is stored as the amount of deviation of the environmental temperature from the reference temperature (for example, 25°C) and the correction amount and the correction ratio (correction coefficient) corresponding thereto, for example, in a temperature range of 10 to 40°C.

The calculating unit 12 calculates the blood glucose level on the basis of the response current value (or the voltage value obtained by converting the response current value). The calculating unit 12 further performs the calculation in order to correct the response current value (or the voltage value obtained by converting the response current value or the calculated blood glucose level) on the basis of the temperature correction data corresponding to the environmental temperature. In this embodiment, the "response current value (or the voltage value obtained by converting the response current value)" corresponds to the "information with respect to the objective substance contained in the sample" according to the present invention. In this embodiment, the "corrected response current value (or the corrected voltage value or the corrected blood glucose level)" corresponds to the "analysis value" according to the present invention.

The calculating unit 12 may be constructed to calculate the amount of change of the environmental temperature measured by the temperature measuring unit 6 every time when a certain period of time elapses. The time interval for calculating the amount of change of the environmental temperature is, for example, 0.1 second to 3 hours, and preferably 1 second to 180 seconds.

The determining unit 13 determines whether or not the environmental temperature, which is measured by the temperature measuring unit 6, is within a temperature range in which the temperature correction can be appropriately performed (whether or not the environmental temperature is within a temperature range in which the temperature correction data is prepared). For example, when the temperature correction data is prepared within a range of 10 to 40°C, the determining unit 13 determines or discriminates whether or not the environmental temperature, which is measured by the temperature measuring unit 6, is within the range of 10 to 40°C.

Further, the determining unit 13 may be constructed to finally determine whether or not the environmental temperature is within a temperature range in which the temperature correction data is applicable, depending on whether or not the amount of change of the environmental temperature per predetermined time is larger than a predetermined threshold value (for example, 0.01 to 1.0°C/second). That is, the determining unit 13 may determine that the environmental temperature is within the temperature range in which the blood glucose level can be calculated appropriately, if it is determined that the environmental temperature is within the predetermined temperature range and the amount of change of the environmental temperature is not more than the predetermined threshold value. Of course, the determining unit 13 may be constructed to determine whether or not the environmental temperature is within a temperature range in which the temperature correction data is applicable, on the basis of only the amount of change of the environmental temperature per predetermined time.

In this arrangement, each of the control unit 10, the storage unit 11, the calculating unit 12, and the determining unit 13 is constructed by a processing device (for example, CPU or MPU) or a memory device (for example, ROM or RAM). However, all of the control unit 10, the storage unit 11, the calculating unit 12, and the determining unit 13 can be also constructed by combining a plurality of memory devices with respect to one processing device.

The voltage applying unit 14 is provided to apply the voltage to the plurality of electrodes 25 to 28 (terminal portions 25A to 28A) of the biosensor 2 by the aid of the plurality of terminals 42, 43. For example, a DC power source is used as the voltage applying unit 14.

The current measuring unit 15 is provided to measure the response current obtained when the voltage is applied by the voltage applying unit 14 to the plurality of electrodes 25 to 28 (terminal portions 25A to 28A) of the biosensor 2.

Next, an explanation will be made with respect to a blood glucose level measuring method based on the use of the blood glucose level measuring apparatus 1.

When the blood glucose level is measured, the biosensor 2 is firstly installed (attached) to the blood glucose level measuring apparatus 1. In this situation, the blood glucose level measuring apparatus 1 recognizes that the biosensor 2 is installed. On the other hand, the determining unit 13 of the blood glucose level measuring apparatus 1 performs the sampling of the environmental temperature measured by the temperature measuring unit 6 to determine whether or not the environmental temperature is within the temperature range in which the blood glucose level measuring apparatus 1 is usable (within the temperature range in which temperature correction data is generated).

The control unit 10 displays the determination result obtained by the determining unit 13 on the display panel 34 to inform a user whether or not the temperature is such a temperature that the blood glucose level measuring apparatus 1 can appropriately measure the blood glucose level. When the user is informed of the determination result, it is also allowable that the user may be informed of the determination result by means of any voice or any lamp without being limited to the display panel 34.

When the user is informed of the determination result as described above, the user can select whether or not blood is spotted on the biosensor 2 to measure the blood glucose level. That is, in the case of such a state that the blood glucose level cannot be measured appropriately due to the environmental temperature, the user can stop the measurement of the blood glucose level before spotting blood on the biosensor 2.

It is also allowable that the calculating unit 12 is used to calculate the amount of change of the environmental temperature at every constant time, and the determining unit 13 is used to determine whether or not the change of the environmental temperature is smaller than a threshold value as a constant value, even if it is determined by the determining unit 13 that the environmental temperature is within the temperature range in which the blood glucose level measuring apparatus 1 is usable. By doing so, it is possible to grasp the amount of change of the environmental temperature. Therefore, it is possible to calculate the correction value on the basis of the environmental temperature provided when the environmental temperature is stable (scarcely undergoes any change). As a result, it is possible to calculate the appropriate correction amount, and it is possible to calculate the blood glucose level more correctly, even in such a situation that a relatively large temperature change is brought about between the environmental temperature provided at the point in time at which the environmental temperature is firstly determined to be the temperature capable of performing the measurement and the environmental temperature provided during the measurement of the blood glucose level (response current value).

It is not necessarily indispensable that the determination based on the environmental temperature should be performed when the biosensor 2 is installed to the blood glucose level measuring apparatus 1. It is also allowable that the determination based on the environmental temperature is performed, for example, by performing a predetermined operation, for example, such that the user depresses the operation button 33 of the blood glucose level measuring apparatus 1. Of course, even in this case, the user is informed of the determination result obtained by the determining unit 13 on the display panel 34.

The blood glucose level measuring apparatus 1 is provided with the main power source for supplying the electric power. The determination based on the environmental temperature, which is performed by the determining unit 13, may be performed continuously provided that the main power source of the blood glucose level measuring apparatus 1 is turned ON. In this case, the user may be informed of the result obtained by the determining unit 13 on the display panel 34 by performing a predetermined operation, for example, such that the user depresses the operation button 33 of the blood glucose level measuring apparatus 1.

If it is determined in the determining unit 13 that the blood glucose level measuring apparatus 1 is in such a state that the blood glucose level cannot be measured appropriately resulting from the environmental temperature, the blood glucose level measuring apparatus 1 may perform the same or equivalent determination when a certain time elapses. The determination as described above is performed until it is determined that the blood glucose level measuring apparatus 1 is in the state in which the blood glucose level can be measured appropriately or until a predetermined number of trials are completed, the predetermined number being previously determined. It is of course allowable that the determination as described above is performed in the determining unit 13 every time when the user performs a predetermined operation, and the user is informed of the result. If the state, in which the environmental temperature is without the temperature range in which the temperature correction can be performed appropriately, is continued for a certain time, the main power source may be stopped (or the electric power saving mode may be initiated to restrict any predetermined function). It is also allowable that the main power source is automatically started up after a certain time elapses. Accordingly, it is possible to reduce the exhaustion of the battery.

On the other hand, if it is determined in the determining unit 13 that the state is provided such that the blood glucose level can be appropriately measured, it is possible to appropriately perform the measurement of the blood glucose level. Therefore, it is possible to continue the blood glucose level measurement by introducing blood into the capillary 23 from the end portion of the capillary 23 of the biosensor 2 by the user. When blood is supplied to the biosensor 2, then the capillary 23 is filled with blood, and the reagent layer 24 is dissolved to construct the liquid phase reaction system. In this situation, when the voltage is applied by the voltage applying unit 14 between the working electrode and the counter electrode of the plurality of electrodes 25 to 28, then glucose, which is contained in blood, is oxidized (electrons are taken out) by the oxidoreductase, and the electrons are supplied to the working electrode via the electron transport (electron transfer) substance. The amount of electrons supplied to the working electrode is measured as the response current by the current measuring unit 15 by the aid of the terminal 42 (43) of the connector unit 4. In the blood glucose level measuring apparatus 1, the glucose concentration (blood glucose level) is calculated by the calculating unit 12 on the basis of the response current mentioned above and the temperature correction data corresponding to the environmental temperature. The calculation result of the blood glucose level is displayed on the display panel 34 by the control unit 10. In this embodiment, the "working electrode", the "counter electrode", and the "reagent layer 24" correspond to the "portion which outputs the information with respect to the objective substance contained in the sample" according to the present invention.

When the user is informed of the determination result obtained by the determining unit 13, for example, on the display panel 34, it is possible to suppress such a situation that the blood glucose level is measured by the blood glucose level measuring apparatus 1, in spite of the state in which it is impossible to appropriately measure the blood glucose level by the blood glucose level measuring apparatus 1 resulting from the environmental temperature. As a result, it is possible to enhance the reliability and the reproducibility in relation to the measurement result of the blood glucose level measuring apparatus 1. Further, it is unnecessary to perform the remeasurement. Therefore, it is possible to avoid any useless use of the biosensor 2.

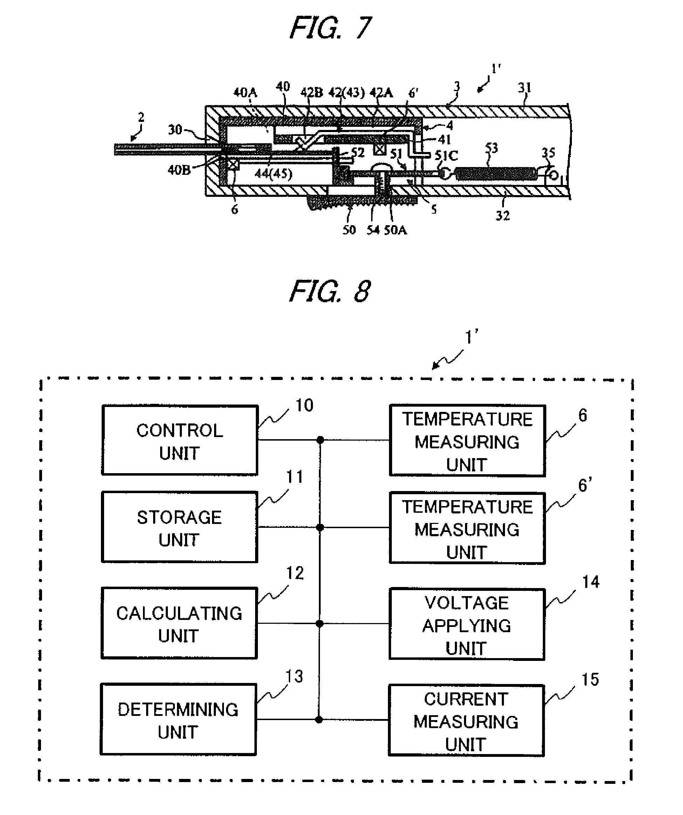

Next, another example of a blood glucose level measuring apparatus 1' will be explained with reference to Figs. 7 and 8. However, in Figs. 7 and 8, the elements, which are the same as or equivalent to those of the blood glucose level measuring apparatus 1 having been explained previously with reference to Figs. 1 to 6, are designated by the same reference numerals. Any duplicated explanation will be omitted from the following description.

The blood glucose level measuring apparatus 1' illustrated in Figs. 7 and 8 is provided with a temperature measuring unit 6' in addition to the temperature measuring unit 6. The determining operation, which is performed in a determining unit 13', is different from that of the blood glucose level measuring apparatus 1 explained above with reference to Figs. 1 to 6.

The temperature measuring unit 6' is provided to measure the internal temperature of the blood glucose level measuring apparatus 1' (housing 3). The temperature measuring unit 6' is provided at a portion which is relatively separated from the reagent layer 24 when the biosensor 2 is installed to the blood glucose level measuring apparatus 1'. Those usable as the temperature measuring unit 6' include, for example, a thermistor as well as various known devices, in the same manner as the temperature measuring unit 6.

The determining unit 13' determines whether or not the environmental temperature, which is measured by the temperature measuring unit 6, is within the temperature range in which the temperature correction data is applicable. For example, when the temperature correction data is prepared within a range of 10 to 40°C, the determining unit 13' discriminates whether or not the environmental temperature, which is measured by the temperature measuring unit 6, is within a range of 10 to 40°C.

Further, the determining unit 13' is constructed to finally determine whether or not the environmental temperature is within the temperature range in which the temperature correction data is applicable, depending on whether or not the difference between the environmental temperature measured by the temperature measuring unit 6 and the environmental temperature measured by the temperature measuring unit 6' is larger than a preset or predetermined threshold value (for example, 0.5 to 5.0°C). That is, the determining unit 13' is constructed to determine that the blood glucose level can be appropriately calculated at the present environmental temperature, if it is determined that the environmental temperature is within the predetermined temperature range, and the difference between the environmental temperatures measured by the two temperature measuring units 6, 6' is not more than the threshold value.

When the determining method as described above is adopted, it is possible to measure the blood glucose level when the reagent layer 24 of the biosensor 2 sufficiently approaches the environmental temperature at the measurement place and the internal temperature of the blood glucose level measuring apparatus 1', if the difference between the internal temperature of the blood glucose level measuring apparatus 1' and the temperature of the reagent layer 24 of the biosensor 2 is large, for example, if the difference in the environmental temperature between the inside and the outside of the blood glucose level measuring apparatus 1' is large as in such a case that the blood glucose level measuring apparatus 1' and the biosensor 2 are moved to any place having any different environmental temperature to measure the blood glucose level. Therefore, it is possible to enhance the reliability and the reproducibility of the measurement result obtained by the blood glucose level measuring apparatus 1'.

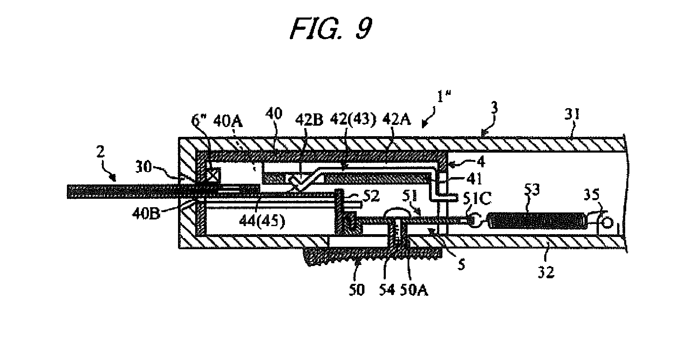

The present invention is not limited to the embodiments explained above, which can be changed in various ways. For example, in the blood glucose level measuring apparatuses 1, 1' illustrated in Figs. 2 and 8, the temperature measuring unit 6 is arranged so that the environmental temperature is measured from the lower surface side of the biosensor 2. However, as in a blood glucose level measuring apparatus 1" illustrated in Fig. 9, a temperature measuring unit 6" may be arranged so that the environmental temperature is measured from the upper surface side of the biosensor 2.

Next, an explanation will be made with reference to the drawings with respect to a glucose continuous measuring apparatus 7 as still another example of the analysis apparatus and the analysis method according to the present invention.

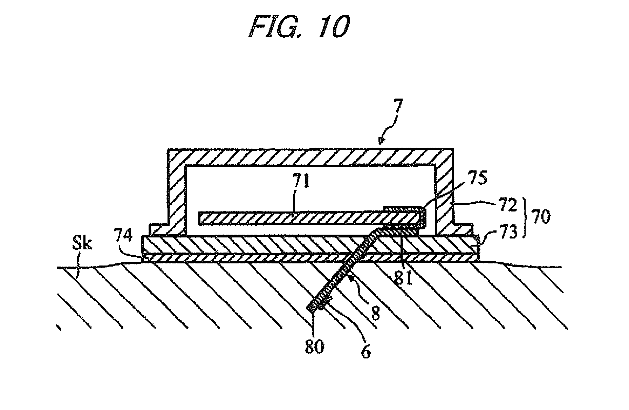

The glucose continuous measuring apparatus 7 illustrated in Fig. 10 is capable of continuously measuring the glucose concentration in the body fluid such as blood, interstitial fluid or the like. The glucose continuous measuring apparatus 7 is used while being installed to the skin of the shoulder or the belly (abdomen) of the human body. However, the installation place is not limited thereto, and it is also allowable to install the glucose continuous measuring apparatus 7 to any other portion. The glucose continuous measuring apparatus 7 is provided with a housing 70, a circuit board 71, a biosensor 8, and a temperature measuring unit 6.

The housing 70 forms the outer shape of the glucose continuous measuring apparatus 7, which includes a cover 72 and a substrate 73. The cover 72 and the substrate 73 are provided to accommodate the circuit board 71 in the space defined thereby, and the cover 72 and the substrate 73 are fixed to one another. It is preferable that the housing 70 has the waterproof performance or the water resistant performance. The housing 70 as described above is constructed, for example, such that at least the cover 72 (and the substrate 73, if necessary) is formed of a material such as metal, polypropylene resin or the like having the extremely low water permeance.

The substrate 73 is a portion through which the biosensor 8 is inserted, and the substrate 73 fixes an end portion 81 of the biosensor 8. An adhesive film 74 is fixed to the substrate 73. The adhesive film 74 is utilized when the glucose continuous measuring apparatus 7 is fixed to the skin. A tape, which has the adhesive property on the both surfaces, can be used as the adhesive film 74.

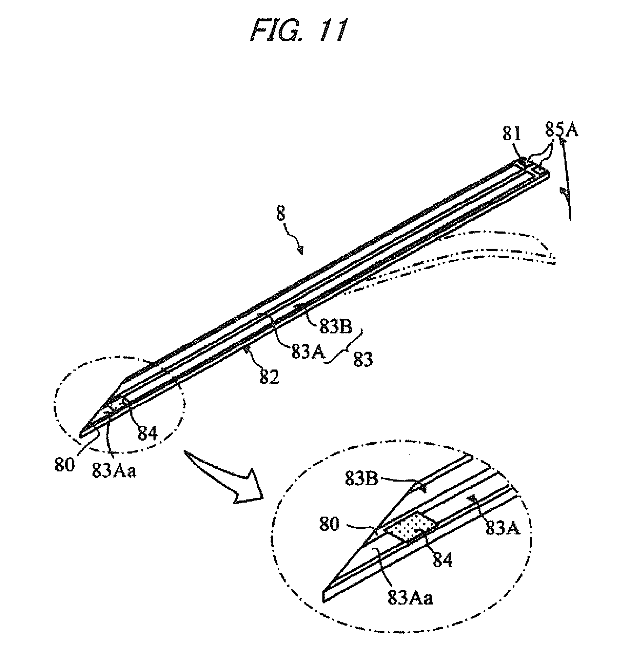

The circuit board 71 carries thereon electronic parts required for predetermined operations of the glucose continuous measuring apparatus 7 (for example, the application of the voltage, the calculation of the glucose concentration, and the communication with the outside).
The circuit board 71 further includes terminals 75 to be brought in contact with electrodes 83 (see Fig. 11) of the biosensor 8 as described later on. The terminals 75 are utilized in order that the voltage is applied to the biosensor 8 and the response current value is obtained from the biosensor 8. In this embodiment, the "response current value" corresponds to the "information with respect to the objective substance contained in the sample" according to the present invention.

The biosensor 8 is provided to obtain the response current corresponding to the glucose concentration in the body fluid such as blood, interstitial fluid or the like. The end portion 81 of the biosensor 8 protrudes from the skin Sk, and the end portion 81 is brought in contact with the terminals 75 of the circuit board 71. Further, greater parts of the biosensor 8 other than the end portion 81 are held (subjected to the detention) in the skin Sk.
In this embodiment, the "glucose concentration in the body fluid such as blood, interstitial fluid or the like" corresponds to the "analysis value" according to the present invention.

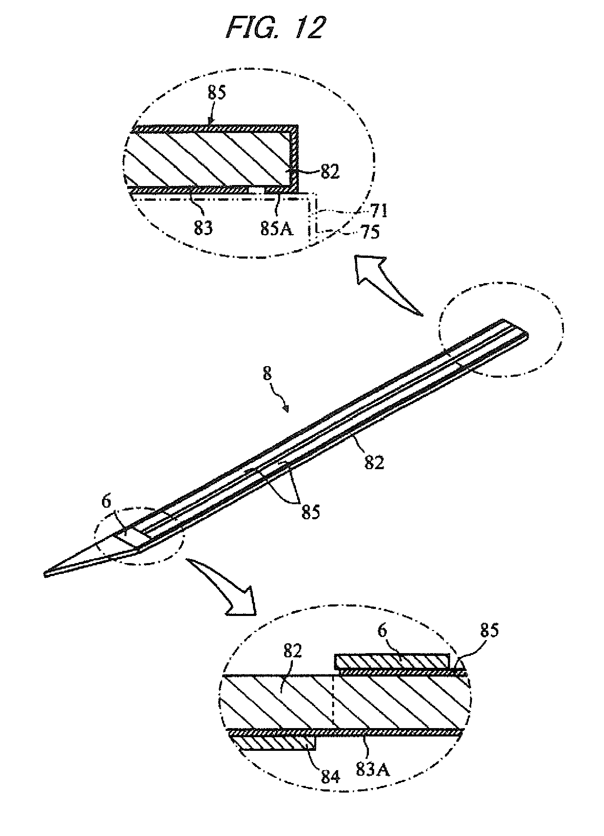

As illustrated in Figs. 11 and 12, the biosensor 8 has a substrate 82, electrodes 83, an immobilized enzyme unit 84, and leads 85.

The substrate 82 is provided to support the electrodes 83. The substrate 82 is formed to have a sheet-shaped form having the insulation performance and the flexibility. The end portion 81 of the substrate 82 exists at the inside of the housing 70, while an end portion 80 is formed to be sharp. When the end portion 80 has the sharp structure, then the biosensor 8 can be easily subjected to the detention in the skin Sk, and it is possible to reduce the pain of a user. The structure of the end portion 80 and the detention method in the skin Sk are not limited thereto. For example, JP2003-527138W describes that a needle is arranged in a cannula to insert the cannula subcutaneously together with the needle, and then the cannula is held (subjected to the detention) subcutaneously by extracting only the needle from the skin. Similarly thereto, a needle is arranged in the biosensor 8 to insert the biosensor 8 into the skin Sk together with the needle, and then the biosensor 8 can be held (subjected to the detention) in the skin Sk by extracting only the needle from the skin Sk. In this case, it is appropriate that the needle has a sharp structure, and it is also allowable that the end portion 80 of the biosensor 8 does not have any sharp structure. Further, for example, JP2008-506468W describes that a sensor is arranged in a hollow needle to insert the needle into the skin, and then the sensor is held (subjected to the detention) in the skin by extracting only the needle from the skin. Similarly thereto, a hollow needle, in which the biosensor 8 is arranged at the inside, is inserted into the skin, and then the biosensor 8 can be held (subjected to the detention) in the skin Sk by extracting only the needle from the skin Sk. Also in this case, it is appropriate that the needle has a sharp structure, and it is also allowable that the end portion 80 of the biosensor 8 does not have any sharp structure.

It is appropriate that a material, which is not harmful to the human body and which has the appropriate insulation performance, is used for the substrate 82. Those usable as the material include, for example, thermoplastic resins such as PET, PP, PE and the like, and thermosetting resins such as polyimide resins, epoxy resins and the like.

The electrodes 83 are utilized to apply the voltage to the immobilized enzyme unit 84 and take out electrons from the immobilized enzyme unit 84. The electrodes 83 are formed on the upper surface of the substrate 82, and the electrodes 83 include a working electrode 83A and a counter electrode 83B. The working electrode 83A is the portion at which the electrons are delivered/received with respect to glucose. The counter electrode 83B is utilized to apply the voltage together with the working electrode 83A. The electrodes 83 can be formed by means of the screen printing based on the use of a carbon ink.

The immobilized enzyme unit 84 mediates the electron delivering/receiving action between glucose and the working electrode 83A by the aid of the enzyme or the electron transport (transfer) substance. The immobilized enzyme unit 84 is formed by immobilizing glucose oxidoreductase to the end portion 83Aa of the working electrode 83A on the upper surface of the substrate 82.

Those usable as the glucose oxidoreductase include glucose oxidase (GOD) and glucose dehydrogenase (GDH). Those adoptable as the method for immobilizing the glucose oxidoreductase include various known methods, for example, any method to utilize polymerizable gel, high molecular weight compound such as polyacrylamide, phosphorus or the like, MPC polymer comprising silane coupling agent introduced into phospholipid polymer, or protein film.

The leads 85 are provided to transmit the information measured by the temperature measuring unit 6 to the circuit board 71. Greater parts of the leads 85 are formed on the lower surface of the substrate of the biosensor 8. One end portion of the lead 85 is brought in contact with the temperature measuring unit 6, while the other end portion of the lead 85 is exposed on the upper surface of the biosensor 8.

The temperature measuring unit 6 is provided to measure the environmental temperature. The temperature measuring unit 6 is provided at a position corresponding to the immobilized enzyme unit 84 on the lower surface of the substrate 82 of the biosensor 8 so that the temperature measuring unit 6 can measure the temperature in the vicinity of the immobilized enzyme unit 84 of the biosensor 8, i.e., the subcutaneous temperature of the human body. The temperature measuring unit 6 is brought in contact with the terminals 75 of the circuit board 71 at the end portion 85A via the lead 85. Those usable as the temperature measuring unit 6 include, for example, a thermistor as well as various known devices.

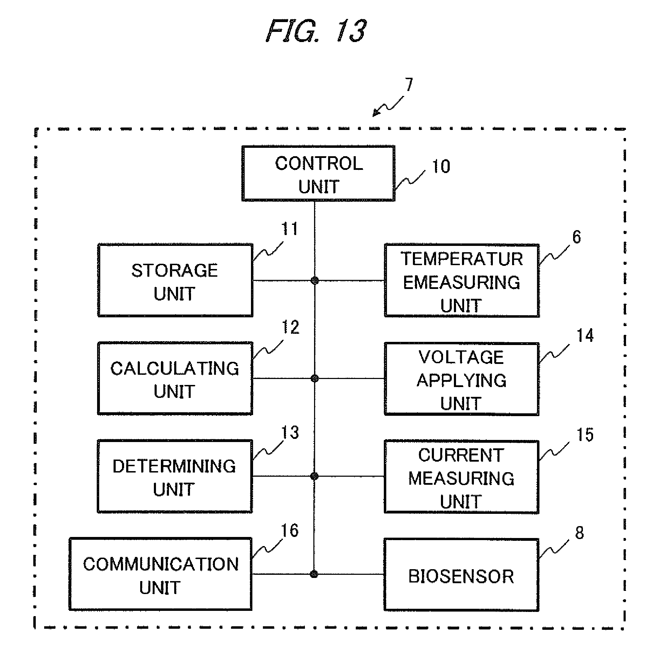

As illustrated in Fig. 13, the glucose continuous measuring apparatus 7 is provided with a control unit 10, a storage unit 11, a calculating unit 12, a determining unit 13, a voltage applying unit 14, a current measuring unit 15, and a communication unit 16, in addition to the circuit board 71, the biosensor 8, and the temperature measuring unit 6.

The control unit 10, the storage unit 11, the calculating unit 12, the determining unit 13, the voltage applying unit 14, and the current measuring unit 15 are the same as or equivalent to the control unit 10, the storage unit 11, the calculating unit 12, the determining unit 13, the voltage applying unit 14, and the current measuring unit 15 of the blood glucose level measuring apparatus 1 illustrated in Fig. 6 explained above, in relation to the respective functions.

The communication unit 16 is provided to perform the data communication between the glucose continuous measuring apparatus 7 and an external information processing terminal. The communication unit 16 has at least a transmitting unit, and the communication unit 16 includes a receiving unit, if necessary.

For example, any wireless communication means (IrDA based on the use of the infrared ray or Bluetooth based on the use of the frequency band of 2.4 GHz) can be utilized for the data communication. It is of course allowable that the data communication is performed in a wired manner by using a cable or the like between the communication unit 16 of the glucose continuous measuring apparatus 7 and the communication unit of the external information processing terminal.

The external information processing terminal can be exemplified, for example, by an insulin feeding device for administering insulin to the human body, a simple type blood glucose level measuring device, a watch type indicator, or a personal computer.

In the glucose continuous measuring apparatus 7, the environmental temperature (subcutaneous temperature), which is measured by the temperature measuring unit 6, is sampled (subjected to the sampling) by the determining unit 13 to determine whether or not the environmental temperature is within a temperature range in which the glucose continuous measuring apparatus 7 is usable (temperature range in which the temperature correction data is generated).

The control unit 10 displays the determination result obtained by the determining unit 13 on the informing means such as a display panel or the like, and/or the control unit 10 transmits the determination result to the external information processing terminal by the aid of the communication unit 16. When the determination result is transmitted to the external information processing terminal, the external information processing terminal informs the user of the determination result.

Even when it is determined by the determining unit 13 that the environmental temperature is within the temperature range in which the glucose continuous measuring apparatus 7 is usable, then the amount of change of the environmental temperature at every constant time may be calculated by the calculating unit 12, and it may be determined by the determining unit 13 whether or not the change of the environmental temperature is smaller than a threshold value. It is of course allowable that the determining unit 13 determines that the environmental temperature is within the temperature range in which the glucose continuous measuring apparatus 7 is usable, on the basis of only the determination to determine whether or not the change of the environmental temperature is smaller than the threshold value.

If it is determined in the determining unit 13 that the state is provided to appropriately measure the glucose concentration in the body fluid such as blood, interstitial fluid or the like, the response current, which is obtained when the voltage is applied by the voltage applying unit 14 between the working electrode 83A and the counter electrode 83B, is measured by the current measuring unit 15. In the case of the determination that the environmental temperature is within the temperature range in which the glucose continuous measuring apparatus 7 is usable, the glucose concentration is calculated by the calculating unit 12 on the basis of the temperature correction data corresponding to the environmental temperature and the response current as explained above. The user is informed of the calculation result of the glucose concentration in the body fluid such as blood, interstitial fluid or the like on the informing means such as the display panel by means of the control unit 10, and/or the calculation result is transmitted to the external information processing terminal by the aid of the communication unit 16. In this embodiment, the "working electrode 83A", the "counter electrode 83B", and the "immobilized enzyme unit 84" correspond to the "portion which outputs the information with respect to the objective substance contained in the sample" according to the present invention.

On the other hand, it is also allowable that the voltage is not applied between the working electrode 83A and the counter electrode 83B, if it is determined in the determining unit 13 that the environmental temperature is not within the temperature range in which the glucose continuous measuring apparatus 7 is usable (such a state is provided that the glucose concentration in the body fluid such as blood, interstitial fluid or the like cannot be measured appropriately). That is, it is also allowable that the voltage is applied between the working electrode 83A and the counter electrode 83B only when such a state is provided that the glucose concentration in the body fluid such as blood, interstitial fluid or the like can be measured appropriately. It is also allowable that it is determined again whether or not the environmental temperature is within the temperature range in which the glucose continuous measuring apparatus 7 is usable, for example, after a certain or constant time elapses after the stop of the voltage application between the working electrode 83A and the counter electrode 83B while it is determined that the environmental temperature is not within the temperature range in which the glucose continuous measuring apparatus 7 is usable. Further, the glucose continuous measuring apparatus 7 is provided with the main power source to supply the electric power. Therefore, for example, when the state, in which the environmental temperature is not within the temperature range in which the glucose continuous measuring apparatus 7 is usable, is continued for a certain time, it is also allowable that the main power source is stopped (or the electric power saving mode, in which predetermined functions are restricted, is initiated). The main power source may be started up automatically after a certain time elapses. Accordingly, it is possible to suppress any useless voltage application between the working electrode 83A and the counter electrode 83B. Therefore, it is possible to suppress the biosensor 8 from being deteriorated. Therefore, it is possible to prolong the service life of the biosensor 8. Further, it is possible to reduce the exhaustion of the battery. When the voltage is continuously applied between the working electrode 83A and the counter electrode 83B, the following procedure is also available. That is, when the environmental temperature is not within the temperature range in which the glucose continuous measuring apparatus 7 is usable (such a state is provided that the glucose concentration in the body fluid such as blood, interstitial fluid or the like cannot be measured appropriately), then the glucose concentration in the body fluid such as blood, interstitial fluid or the like is not calculated, and/or the user is informed that the calculated glucose concentration in the body fluid such as blood, interstitial fluid or the like has any inappropriate value.

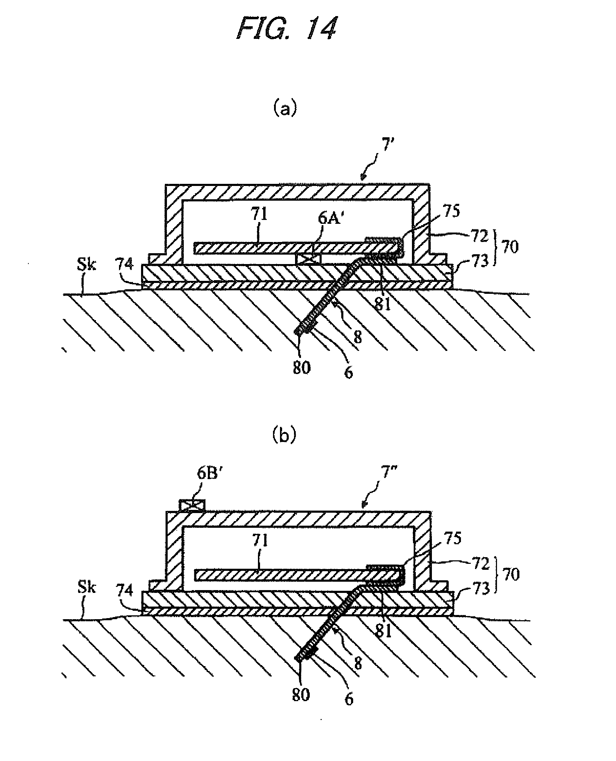

The glucose continuous measuring apparatus 7 explained with reference to Figs. 10 to 13 may be provided with other temperature measuring units 6', 6" as in glucose continuous measuring apparatuses 7', 7" illustrated in Figs. 14(a) and 14(b), in addition to the temperature measuring unit 6, in the same manner as the blood glucose level measuring apparatuses 1', 1" explained with reference to Figs. 7 to 9. In Figs. 14(a) and 14(b), the elements, which are the same as or equivalent to those of the glucose continuous measuring apparatus 7 explained with reference to Figs. 10 to 13, are designated by the same reference numerals. The temperature measuring unit 6' is provided at a portion which is relatively separated from the immobilized enzyme unit 84 in the biosensor 8. The temperature measuring unit 6" is provided on the same surface as that of the substrate 82 formed with the immobilized enzyme unit 84 in the biosensor 8.

The glucose continuous measuring apparatus 7' illustrated in Fig. 14(a) has the temperature measuring unit 6A' which is arranged so that the epidermal temperature can be measured. The temperature measuring unit 6A' is provided between the circuit board 71 and the substrate 73.

The glucose continuous measuring apparatus 7" illustrated in Fig. 14(b) has the temperature measuring unit 6B' which is arranged so that the outside air temperature can be measured. The temperature measuring unit 6B' is provided on the surface of the cover 72.

The glucose continuous measuring apparatuses 7', 7" can be constructed so that the determining unit determines whether or not the temperature is within the temperature range in which the temperature correction data is applicable, on the basis of the difference between the environmental temperature (subcutaneous temperature) which is measured by the temperature measuring unit 6 and the environmental temperature (epidermal temperature or outside air temperature) which is measured by the temperature measuring unit 6A', 6B'. That is, it is also possible to construct the apparatus so that it is determined whether or not the temperature is within the temperature range in which the temperature correction data is applicable, depending on whether or not the difference between the subcutaneous temperature and the epidermal temperature or the outside air temperature is not more than a predetermined threshold value (for example, 0.5 to 5.0°C).

The glucose continuous measuring apparatus 7 explained with reference to Figs. 10 to 13 may be provided with both of the temperature measuring units 6A', 6B' in addition to the temperature measuring unit 6. In this arrangement, the apparatus can be also constructed so that it is determined whether or not the temperature is within the temperature range in which the temperature correction data is applicable, depending on whether or not the value, which is obtained by subtracting both of the environmental temperature (epidermal temperature) measured by the temperature measuring unit 6A' and the environmental temperature (outside air temperature) measured by the temperature measuring unit 6B' from the environmental temperature (subcutaneous temperature) measured by the temperature measuring unit 6, is not more than a predetermined threshold value (for example, 0.5 to 5.0°C). Further, it is also allowable to construct the apparatus as follows. That is, the apparatus individually performs the determination to determine whether or not the difference between the subcutaneous temperature and the epidermal temperature is not more than a predetermined threshold value (for example, 0.5 to 5.0°C) and the determination to determine whether or not the difference between the subcutaneous temperature and the outside air temperature is not more than a predetermined threshold value (for example, 0.5 to 5.0°C). It is determined whether or not the temperature is within the temperature range in which the temperature correction data is applicable, on the basis of one or both of the determination results obtained as described above.

In the glucose continuous measuring apparatus 7 explained with reference to Figs. 10 to 13, the temperature measuring unit, which is provided in addition to the temperature measuring unit 6, may be arranged at any portion different from those of the temperature measuring units 6A', 6B' illustrated in Figs. 14(a) and 14(b).

The present invention is not limited to the blood glucose level measuring apparatus for measuring the blood glucose level and the glucose continuous measuring apparatus, and the present invention is also applicable to other analysis apparatuses. That is, the present invention is applicable to those which analyze any component other than glucose including, for example, cholesterol and lactic acid contained in a sample, as well as those which analyze any specified component by using a sample other than the blood sample.

Further, the present invention is not limited to the analysis apparatus which analyzes a sample by means of the electrochemical technique, and the present invention is also applicable to any analysis apparatus which is constructed to analyze a sample by means of an optical technique.

### Description of the Reference Numerals and Symbols

1, 1', 1'' blood glucose level measuring apparatus (analysis apparatus)
7, 7', 7'' glucose continuous measuring apparatus (analysis apparatus)
11 storage unit (storage means)
12 calculating unit (calculating means)
13, 13' determining unit (determining means)
2, 8 biosensor
34 display unit (informing means)
33 operation buttons
6, 6'' temperature measuring unit (temperature measuring means)
6', 6A', 6B' temperature measuring unit (additional temperature measuring means)

## Claims

1. An analysis apparatus for obtaining information with respect to an objective substance contained in a sample, the analysis apparatus comprising:
temperature measuring means which measures an environmental temperature;
storage means which stores temperature correction data corresponding to the environmental temperature in relation to a predetermined temperature range;
calculating means which calculates an analysis value on the basis of the temperature correction data and the information with respect to the objective substance contained in the sample; and
determining means which determines whether or not the environmental temperature is within the temperature range, wherein:
the determining means determines whether or not the environmental temperature is within the temperature range even in such a situation that the information with respect to the objective substance contained in the sample is not obtained from the analysis apparatus.

2. The analysis apparatus according to claim 1,
wherein the determining means is constructed to determine whether or not the temperature is within a temperature range in which the temperature correction data is applicable, depending on whether or not an amount of change per predetermined time of the environmental temperature measured a plurality of times by the temperature measuring means is within a predetermined range.

3. The analysis apparatus according to claim 1,
wherein the temperature measuring means is provided at a portion at which it is possible to measure a temperature in a vicinity of a portion which outputs the information with respect to the objective substance contained in the sample.

4. The analysis apparatus according to claim 1, further comprising additional temperature measuring means which measures the environmental temperature distinctly from the temperature measuring means.

5. The analysis apparatus according to claim 4,
wherein the determining means is constructed to determine whether or not the temperature is within a temperature range in which the temperature correction data is applicable, on the basis of a difference between the environmental temperature which is measured by the temperature measuring means and the environmental temperature which is measured by the additional temperature measuring means.

6. The analysis apparatus according to claim 4,
wherein the additional temperature measuring means is provided at a portion separated from a portion which outputs the information with respect to the objective substance contained in the sample as compared with the temperature measuring means.

7. The analysis apparatus according to any one of claims 1 to 6, wherein the determining means determines again whether or not the environmental temperature is within a temperature range in which the temperature correction data is applicable, after a certain time elapses, if it is determined that the environmental temperature is without the temperature range in which the temperature correction data is applicable.

8. The analysis apparatus according to claim 7, further comprising:
a main power source, wherein:
the determining means stops the main power source or performs electric power saving after a certain time elapses, if it is determined that the environmental temperature is without the temperature range in which the temperature correction data is applicable.

9. The analysis apparatus according to any one of claims 1 to 8, further comprising informing means which informs that the environmental temperature is within the temperature range in which the temperature correction data is applicable or that the environmental temperature is without the temperature range in which the temperature correction data is applicable, on the basis of a determination result obtained by the determining means.

10. The analysis apparatus according to any one of claims 1 to 9, wherein an apparatus, which can be held in a body and which is capable of continuously analyzing the sample, is used as the analysis apparatus.

11. An analysis method for obtaining information with respect to an objective substance contained in a sample, the analysis method comprising:
a temperature measuring step of measuring an environmental temperature;
a storing step of storing temperature correction data corresponding to the environmental temperature in relation to a predetermined temperature range;
a calculating step of calculating an analysis value on the basis of the temperature correction data and the information with respect to the objective substance contained in the sample; and
a determining step of determining whether or not the environmental temperature is within the temperature range, wherein:
it is determined in the determining step whether or not the environmental temperature is within the temperature range even in such a situation that the information with respect to the objective substance contained in the sample is not obtained from the analysis step.

12. The analysis method according to claim 11,
wherein it is determined in the determining step whether or not the temperature is within a temperature range in which the temperature correction data is applicable, depending on whether or not an amount of change per predetermined time of the environmental temperature measured a plurality of times in the temperature measuring step is within a predetermined range.

13. The analysis method according to claim 11,
wherein a temperature is measured in a vicinity of a portion which outputs the information with respect to the objective substance contained in the sample in the temperature measuring step.

14. The analysis method according to claim 11, further comprising an additional temperature measuring step of measuring the environmental temperature distinctly from the temperature measuring step.

15. The analysis method according to any one of claims 11 to 14, wherein it is determined again in the determining step whether or not the environmental temperature is within a temperature range in which the temperature correction data is applicable, after a certain time elapses, if it is determined that the environmental temperature is without the temperature range in which the temperature correction data is applicable.
